# EUROPEAN PATENT SPECIFICATION

(11) **EP 0 521 166 B1**
(45) Date of publication and mention of the grant of the patent: **07.04.1999**
(21) Application number: 92903730.7
(22) Date of filing: 23.01.1992
(51) Int. Cl.: C12N 1/20, A23C 9/127

(54) **LACTIC ACID BACTERIUM STARTER, CONTAINING PEROXIDASE, FERMENTED MILK PRODUCT, AND PRODUCTION THEREOF**
PEROXIDASE ENTHALTENDE STARTER-KULTUR EINES MILCHSÄUREBAKTERIUMS, FERMENTIERTES MILCHPRODUKT UND SEINE HERSTELLUNG
FERMENT A BASE DE BACTERIES PRODUISANT L'ACIDE LACTIQUE ET CONTENANT DE LA PEROXIDASE, PRODUIT LAITIER FERMENTE, ET PRODUCTION DUDIT PRODUIT

(30) Priority: 23.01.1991 JP 22927/91
(43) Date of publication of application: 07.01.1993
(73) Proprietor: SNOW BRAND MILK PRODUCTS CO., LTD., Sapporo-shi, Hokkaido 065 (JP)
(72) Inventor: DOSAKO, Shunichi 5-15-39-616, Kitaurawa, Saitama 336 (JP); MITSUHASHI, Shigeyuki, Tanashi-shi Tokyo 188 (JP); SUGIURA, Masayuki 100-6, Nakamachi, Tokyo 187 (JP); HAYASHI, Shigeru Naracho Danchi 11-501, Omiya-shi Saitama 331 (JP); MOCHIZUKI, Eisuke Higashiarai Danchi 3-201, Omiya-shi Saitama 330 (JP); TOYODA, Shuji Rengakan 11-202 3-12-5, Midoricho, Saitama 359 (JP)
(74) Representative: Davies, Jonathan Mark
(86) International application number: JP9200053
(87) International publication number: WO9213064

(56) References cited:
- JP-A-62 228 224
- JP-A-63 226 243
- MILCHWISSENSCHAFT. vol. 45, no. 10, 1990, MUNCHEN DE pages 638 - 641 M.A. EL-SHENAWY ET AL. 'Inhibition and inactivation of Listeria monocytogenes by the lactoperoxidase system in raw milk, buffer or a semi-synthetic medium'
- Bergey's Manual of Systematic Bacteriology, vol.2, page 1243

## Description

### FIELD OF TECHNOLOGY

The present invention relates to a fermented milk product exhibiting a high effect of intestinal function control and an effect of suppressing the acidity increase during storage and transportation (hereinafter referred to as "storage"), to a process for manufacturing such a fermented milk product, and to a lactic acid bacterium starter used for the manufacturing process.

### BACKGROUND ART

Peroxidase (hereinafter called PO) is known to exhibit antibacterial actions against gram negative bacteria, such as *E. coli* and the like, by giving damages to their cell membranes in the presence of thiocyanate and hydrogen peroxide. It exhibits bacteriostatic actions against gram positive bacteria, such as lactic acid bacteria and the like, by temporarily suppressing their growth. Taking the advantage of these characteristics of PO, a number of trials have been undertaken to extend a storage period of foods by the addition of PO together with thiocyanate and hydrogen peroxide.

Since lactic acid bacteria, which are used as a starter in the manufacture of fermented milk and cheese, produce hydrogen peroxide, milk products containing lactic acid bacteria are reported to effectively keep their qualities, if thiocyanate is added in a small amount [J. Food Protection, 47, 724-732 (1984)]. A process for manufacturing food products fermented by lactic acid bacteria, which is characterized by adding PO, thiocyanate, and/or a halogen ion, and by being stored at a temperature below 10°C, for the purpose of extending the storage period by preventing their bacterial deterioration and for the further purpose of removing mutants, is known (Japanese Patent Laid-open (ko-kai) No. 228224/1987).

Even though such a process for the manufacture of fermented milks aims, as one of its targets, at extension of their storage period, there are hardly the case where the bacterial deterioration causes practical problems in developed countries where refrigerators are in popular use. In addition, thiocyanate cannot be added to fermented milks in practice, since it is not a permitted food additive. Furthermore, addition of PO after completion of fermentation according to general practice cannot produce a homogeneous mix, if added to products such as yoghurt having a gelled state.

Lactoperoxidase (hereinafter called LPO), on the other hand, is reported to kill 90% or more of pathogenic *E. coli* orally given to calf, when LPO was administered to the calf at a dose of 19 mg per day [Ann. Rech. Vet., 21, 143-152 (1990)]. In this experiment, in order to enhance the effect of LPO, thiocyanate of an amount equivalent to LPO is added together with glucose and glucoseoxygenase which generates hydrogen peroxide. Since thiocyanate is contained in saliva and gastric juice and hydrogen peroxide is produced by lactic acid bacteria which are present in intestine, administration of LPO alone is considered to be effective.

Fermented milks, including yoghurt, lactic acid bacterium drinks, and their analogs are generally manufactured by preparing a raw material mixture, of which the major component is animal milk, by inoculating to the mixture traditional dairy lactic acid bacteria, such as *Lactobacillus delbrueckii* subsp. *bulgaricus* (hereinafter called *L. bulgaricus*), *Streptococcus salivarius* subsp. *thermophilus* (hereinafter called *S. thermophilus*), *Lactobacillus helveticus* (hereinafter called *L. helveticus*), and *Lactococcus cremoris* (hereinafter called *L. cremoris*), singly or in combination with intestinally useful bacteria, such as *Lactobacillus acidophilus* (hereinafter called *L. acidophilus*), *Bifidobacterium* species, and the like, as a starter, and by fermenting the milk.

These lactic acid bacteria are reported to have various physiological effects themselves. Because of this, intake of living lactic acid bacteria such as fermented milks is expected not only to bring about the physiological effects of lactic acid bacteria, but also to produce intestinal bacterial flora in which lactic acid bacteria are predominant, leading to effects of controlling intestinal functions, e.g., improvement of feces characteristics, etc. Suppression of *E. coli* in the intestine or pathogenic *E. coli* coming from outside may further promote the effect of lactic acid bacteria on the control of intestinal function.

Flavors of fermented milks are affected by organic acids (lactic acid, acetic acid, etc.) aroma compounds (acetaldehyde, diacetyl, acetoin, etc.), and sugars (lactose, monosaccharides, etc.), produced by lactic acid bacteria, and by sweeteners, stabilizers, flavors, and the like which are added to the raw material mixture. The most important factor for all types of fermented milks to be tasty and flavorful is to exhibit a suitable acidic taste, especially, derived from organic acids such as lactic acid and the like.

All the above-mentioned lactic acid bacteria, however, are of the lactose-fermenting type. They ferment lactose contained in the products even under the chilled transportation conditions at 10°C or lower, producing lactic acid and acetic acid which increase acidity of the products and impair their flavor. Although commercial products are usually sold with an indication of a 2-week relish period, their acidity becomes so strong after 1 week that they are hardly relishing, or their quality, i.e., the acidity, greatly changes during the relish period. This tendency is particularly conspicuous in fermented milks containing *Lactobacillus* such as *L. helveticus, L. bulgaricus, L. acidophilus*, or the like.

Heretofore, trials have been undertaken to control this phenomenon of increase in acidity due to excessive acid fermentation. Methods so far reported are as follows.
(1) A method of using low temperature sensitive mutants (Japanese Patent Laid-open (ko-kai) No. 239/1987), and a method of using artificial mutants with no capability of fermenting lactose (Japanese Patent Laid-open (ko-kai) No. 38187/1979 and Japanese Patent Publication (ko-koku) No. 27293/1967).
(2) A method of controlling the acidity increase through control of the number of living bacteria by heating the products for a specified period of time under temperature conditions in which not all bacteria are completely killed [Japanese Patent Laid-open (ko-kai) No. 67451/1975, Milchwissenschaft, 42, 146-148 (1987)].
(3) A method of using a low-lactose skim milk from which lactose has been removed, as a raw material [J. Food Sci., 38, 796-798 (1973)].

Among the above methods, method (1) has a limitation that it is effective only when a specific mutant is used. In addition, the use of artificial mutants in products require a social consensus. Furthermore, flavor, properties, and body of the product obtained are sometimes rather inferior. Thus, the products are not practically usable, even if they possess a suitable acid-producing capability.

In the above method (2), the heat treatment process for a specified period of time at a specified temperature require a complicated procedure. The temperature control is also difficult, requiring energy for heating, and, in addition, heating has a defect that it tends to induce deterioration of product qualities, such as flavor, body, and the like.

The above method (3) is not economically practicable, since it requires a process for removing lactose from raw milks.

Milchwissenschaft 45 (10) 1990 discloses culture of listeria monocytogenes in media containing lactoperoxidase and thiocyanate ions. The procedure included addition of lactoperoxidase to a semisythnetic medium (SSM) and a buffer solution. Thiocyanate ions, pathogen and H₂O₂ were added to the SSM and buffer and a solution of raw milk. They were all incubated at 4 or 35°C and samples were withdrawn periodically to investigate the growth of listeria monocytogenes.

JP-A63-226243 discloses the use of peroxidase to remove mutagenic material from raw milk and milk products such as powdered milk and yoghurt.

The present inventors have conducted extensive studies in order to develop fermented products which can maintain their flavor persistently over the relish period, not by suppressing bacterial deterioration for the purpose of extension of the storage period, but by controlling the increase in the acidity during chilled storage, and can further exhibit an enhanced effect of controlling intestinal functions in living bodies. The studies have led to the completion of the present invention.

### DISCLOSURE OF THE INVENTION

The present invention provides fermented milk products which exhibit an increased effect of controlling intestinal functions, which is peculiar to fermented milks, and of which the increase in acidity and the deterioration of flavor during storage are prevented, as well as a process for manufacturing such fermented milk products. The present invention further provides lactic acid bacterium starters used in the process for manufacturing said fermented milk products.

The feature of the present invention resides in adding PO to a lactic acid bacterium culture and inoculating the culture in a milk medium to cultivate it into fermented milk products. Ideal milk media used in this instance are those not containing PO and thiocyanate.

Commercial horseradish PO, PO extracted from plants, and the like can be used as PO in the present invention. However, since the products are milk products, use of purified LPO separated from milk is desirable. LPO is separated from skim milk or whey during a process step wherein lactoferin is separated from skim milk or whey (Japanese Patent Laid-open (ko-kai) No. 109400/1991).

PO in the present invention includes such LPOs.

The above-mentioned *S. thermophilus, L. bulgaricus, L. helveticus, L. acidophilus,* or *Bifidobacterium* species, and the like, are given as examples of commonly used lactic acid bacteria used as a starter. Mixtures of these bacteria can also be used. They are commercially sold and readily available.

Known methods are applicable to the preparation of starter cultures. Namely, milk, e.g., skim milk powder, is rehydrated with the addition of water, heated to sterilize, cooled, and fermented with the addition of a prescribed amount of PO and inoculation of the above-mentioned lactic acid bacteria; or alternatively, the fermentation may be carried out by inoculating the above-mentioned lactic acid bacteria into a milk media which has been heated and sterilized, followed by the addition of a prescribed amount of PO. The target level of fermentation is to bring the acidity of lactic acid to 0.8-1.5% or the pH to 4.0-4.5. When these conditions are achieved, the culture is cooled to 10°C or lower. It is important in this procedure to add PO to the starter. Japanese Patent Laid-open (ko-kai) No. 228224/1987 describes that if lactic acid bacterium drinks or fermented milks are prepared by using milks wherein LPO has not been inactivated by low temperature pasteurization, the fermentation is unduly retarded due to the competitive inhibition between lactic acid bacteria and LPO, leading eventually to the inactivation of LPO, and hence the process cannot be a usable system. This occurs, however, in systems to which thiocyanate is added or wherein the content of thiocyanate is high. However, if LPO is added to a lactic acid bacterium starter in advance without the addition of thiocyanate as in the process of the present invention, pasteurized milks, substantially not containing thiocyanate or containing an extremely small amount of thiocyanate (less than 0.1 ppm), can be fermented without retardation, as confirmed by the present inventors as shown in the following Table 1.

**TABLE 1**

| | | | Time (hr) | | |
|---|---|---|---|---|---|
| Starter | Media | Amount of LPO added | 0 | 12 | 16 |
| *L. bulgaricus* + *S. thermophilus* | A | 0% | 0.15 | 1.24 | 1.43 |
| | A | 0.01% | 0.14 | 1.19 | 1.34 |
| | A | 0.10% | 0.12 | 1.16 | 1.33 |
| | A | 1.00% | 0.18 | 1.10 | 1.27 |
| *L. acidophilus* + *S. thermophilus* | B | 0% | 0.21 | 1.04 | 1.16 |
| | B | 0.01% | 0.22 | 1.06 | 1.18 |
| | B | 0.10% | 0.24 | 1.07 | 1.18 |
| | B | 1.00% | 0.26 | 1.12 | 1.20 |
| *B. longum* STB-2928 + *B. longum* STB-2933 | C | 0% | 0.25 | 1.19 | 1.57 |
| | C | 0.01% | 0.24 | 1.24 | 1.60 |
| | C | 0.10% | 0.24 | 1.25 | 1.60 |
| | C | 1.00% | 0.28 | 1.24 | 1.58 |
| Medium A: 12% skim milk, sterilized at 95°C for 1 hour. | | | | | |
| Medium B: 11.53% skim milk + 0.5% yeast extract, sterilized at 95°C for 20 minutes. | | | | | |
| Medium C: 11.53% skim milk + 0.5% yeast extract + 0.1% sodium ascorbate, sterilized at 95°C for 2 hours. | | | | | |
| Culturing temperature: 32°C | | | | | |
| Thiocyanate concentration in media: 0.1 ppm or lower in all media (ion chromatography method) | | | | | |
| LPO concentration in raw material media: 25 ng/ml or lower in all media (enzyme immunoassay). | | | | | |

Benefits of adding PO to starters are as follows. (1) In the agitation-type yoghurt, it is difficult to obtain a homogeneous mixture by a conventional pulverizer, even if PO is added when gel is pulverized. The manufacturing facility must be altered by introduction of a homogenizer. In the settlement-type yoghurt, it is difficult to add PO after the completion of fermentation, because the fermentation is carried out after filling milks into containers. (2) PO cannot be pasteurized in advance, since it is unstable to heat. The addition of PO or LPO to sterilized milks may be possible. However, since PO and LPO cannot be pasteurized, the sterilized milks may be contaminated by extraneous bacteria and such bacteria may grow before the pH is lowered by a starter. However, extraneous bacteria cannot grow in starters, since lactic acid bacteria are predominant in the starters. Moreover, fermentation of starters and fermentation of milks to which a starter is inoculated are affected not at all, since *E. coli* and the like will be killed if the pH is successively lowered.

The amount of PO to be added to a starter is determined by calculation taking the PO concentration of the final product into consideration. For example, if the PO concentration of the final product is 50 mg/l and the ratio of the starter culture to be added into milk mixture is 5%, the amount of PO to be added to the starter is 0.1%.

A preferable PO concentration in the final product is 10 mg/l or more when a greater effect on the control of intestinal function is intended. If the amount is smaller than that, the effect on the control of intestinal function can hardly be promoted. There are no specific limitations as to the upper limit of the concentration, although an amount more than 500 mg/l exhibits no remarkable effect on the control of intestinal function proportionate to the added amount; not only the effect remains the same but also color glossiness becomes dull, so that the addition exceeding 500 mg/l brings about no substantial benefit.

The effect of controlling the acidity increase during the chilled storage is manifest at a PO concentration of 3 mg/l or more, even though a concentration of 1 mg/l in the final product has been proven to be effective.

In the preparation of a starter culture, as mentioned above, skim milk powder is usually rehydrated with water, sterilized under heating at 90-95°C for 15-30 minutes, and cooled to 25-45°C, following which bacteria are inoculated. Usually, the milk is cooled to 10°C or lower when the acidity of lactic acid reaches 0.8-1.5% or pH becomes 4.0-4.5. PO may be added at any time after rehydrated skim milk has been sterilized and cooled to 25-45°C, although it is desirable that PO be added just before the milk is cooled to lower than 10°C after the completion of fermentation, when PO powder contains a certain number of bacteria. The reason is that most of the extraneous bacteria will be killed at the completion of fermentation when the starter has a pH of 4.0-4.5. The addition immediately before cooling below 10°C is more advantageous because stirring before cooling is easier, although the addition after cooling below 10°C is acceptable.

A starter culture obtained in this manner is then inoculated into sterilized milk. Raw milks frequently used are fresh milks of which the milk fat content is usually standardized to about 3.5%, for the manufacture of yoghurt; creams having a milk fat content of 45%, for the manufacture of sour milk; and skim milks for the manufacture of quark. There are no specific restrictions to the sterilizing conditions; usually raw milks are heated at 90°C for 10 minutes or at 97°C for 15 seconds. The starter culture is inoculated after cooling the milk to the incubation temperature (about 25-45°C, although the temperature varies depending on the product) in an amount of about 1-10% of the milk. Steps following the inoculation, such as filling after inoculation, fermentation, flavoring, cooling, etc., may be carried out according to conventional methods.

Yoghurt, sour cream, quark, fermented milk drinks, and the like are given as examples of fermented products of the present invention.

### BRIEF DESCRIPTION OF THE DRAWINGS

Figure 1 shows changes in the acidity over time of plain yoghurt obtained in Example 7.

Figure 2 shows changes in pH over time of plain yoghurt obtained in Example 7.

Figure 3 shows changes in the acidity over time of sweet yoghurt obtained in Example 8.

Figure 4 shows changes in pH over time of sweet yoghurt obtained in Example 8.

Figure 5 shows changes in the acidity over time of drink yoghurt obtained in Example 9.

Figure 6 shows changes in pH over time of drink yoghurt obtained in Example 9.

### BEST MODE FOR CARRYING OUT THE INVENTION

The present invention is more specifically described by way of examples.

### [Example 1] Preparation of various lactic acid bacterium starters

A 12% rehydrated skim milk was sterilized at 115°C for 20 minutes. After cooling to 37°C, 1.67% of LPO was added. Mother starters were prepared by the addition of 1% of a mixture of *L. bulgaricus* ATCC 11842 and *S. thermophilus* ATCC 14485 or *L. helveticus* JCM 1120, as a seed culture, followed by cultivation at 37°C for 16 hours. Skim milk without the addition of LPO was used as a control. A bulk starter was prepared by sterilizing said 12% rehydrated skim milk medium at 92°C for 20 minutes, inoculating said mother starter at the final concentration of 3%, and cultivating it at 32°C for 16 hours (*L. helveticus* was cultivated 37°C for 16 hours). This bulk starter was inoculated at the final concentration of 3% to a 10% rehydrated skim milk medium sterilized at 92°C for 20 minutes, and cultivated at 43°C for 3 hours to measure the acidity of lactic acid bacteria as an activity test.

Since *L. acidophilus* JCM 1132 and *L. casei* ATCC 393 have a weaker growing capability as seed cultures, they were inoculated in an amount of 3% to a medium of a 12% rehydrated skim milk to which 0.5% of yeast extracts was added, and cultivated at 37°C for 16 hours to obtain a mother starter. The same amount of LPO was added in the same manner as above. Similarly, a rehydrated skim milk without the addition of LPO was served as a control.

Further, a bulk starter was prepared by sterilizing a 12% rehydrated skim milk medium, to which 0.5% yeast extracts was added, at 92°C for 20 minutes, inoculating said mother starter at the final concentration of 3% after cooling to 37°C, and cultivating it at 37°C for 16 hours.

The results of the cultivation is shown in Table 2. To summarize the results, (1) cultivation was not inhibited in any lactic acid bacteria at the stage of preparation of mother starters, even in the case where 1.67% LPO was added; (2) increase in the lactic acid acidity in bulk starters in which these mother starters were used was almost the same as those of controls at the stage of preparation; and (3) activities of bulk starters were not seen to be affected by the addition of LPO.

The above results confirm that the addition of LPO to starters exhibits no influence on the fermentation of products.

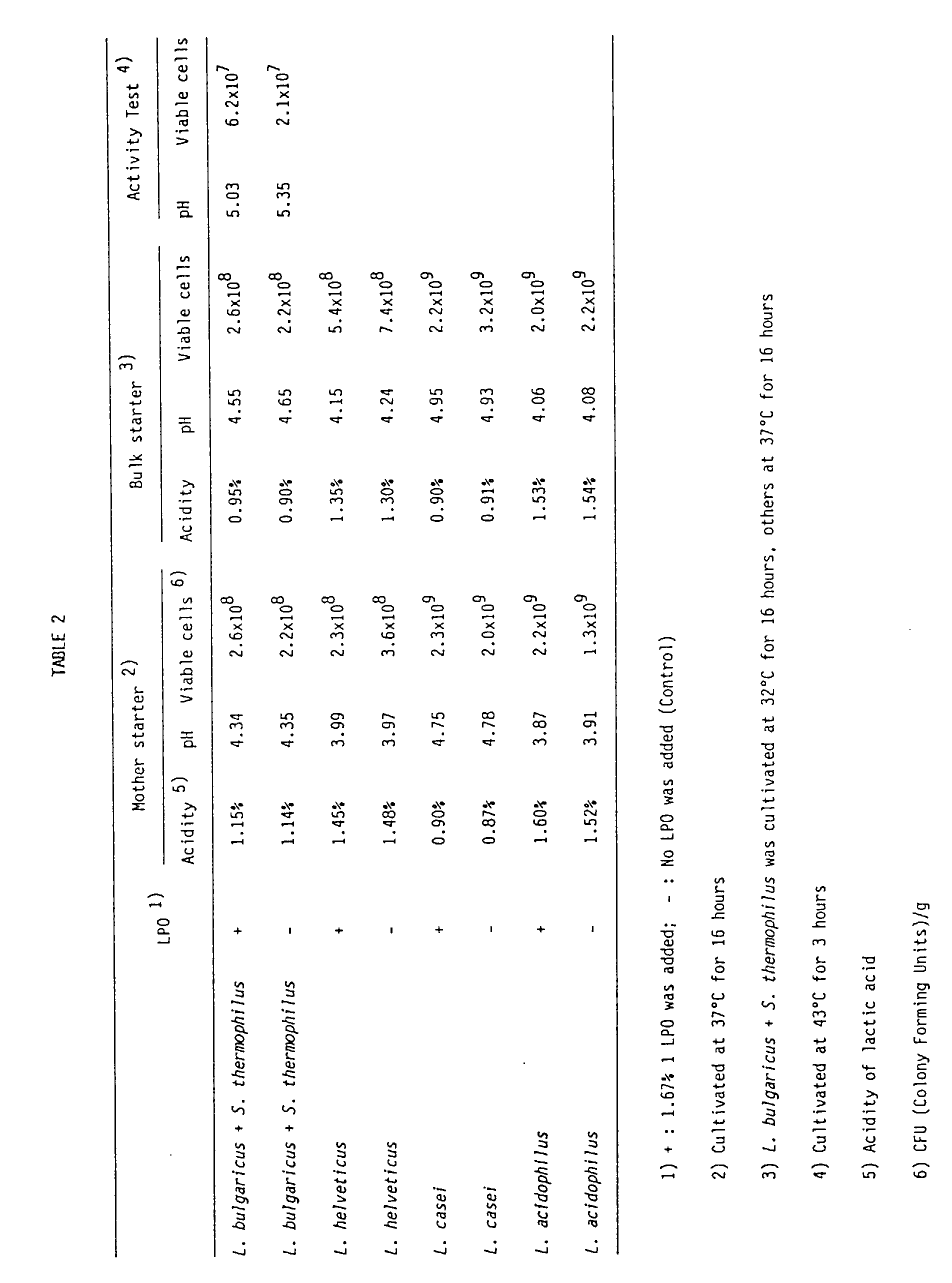

### [Example 2] Preparation of lactic acid bacterium starter

720 g of skim milk powder was dissolved into 5,220 g of water, heated to 95°C and retained at this temperature for 30 minutes to effect sterilization, and cooled to 37°C. Cultivation was carried out at 37°C for 6 hours, after inoculating 60 mg of a commercially available mixed starter consisting of *L. bulgaricus* (Hansen Co.) and *S. thermophilus* (Hansen Co.). The cultivated material was divided into 6 equal portions to prepare 6 lactic acid bacterium starters for yoghurt preparation: (1) without the addition of LPO and with the addition of LPO in an amount of (2) 167 mg, (3) 334 mg, (4) 3345 mg, (5) 16.96 g, and (6) 34.49 g, followed by stirring and cooling.

### [Example 3] Preparation of plain yoghurt

Raw milk (a commercial milk) standardized to a 3.5% milk fat content was maintained at 90°C for 10 minutes to sterilize, and cooled to 37°C. To each 970 g of the raw milk, 30 g of each lactic acid bacterium starter prepared in Example 1 was inoculated, filled in a cup, and cultivated at 37°C until 0.85% lactic acid acidity is achieved, immediately followed by cooling to prepare plain yoghurt. In this manner, plain yoghurts (1)-(6) with different LPO contents were prepared. The results of LPO content measurement, time required for cultivation, and results of the storing test are shown in Table 3.

**TABLE 3**

| | Control | Lactoperoxidase Added | | | | |
|---|---|---|---|---|---|---|
| | (1) | (2) | (3) | (4) | (5) | (6) |
| LPO Concentration (mg/l) | 0 | 4.95 | 10.01 | 98.7 | 498.6 | 998.4 |
| Cultivation time (minutes) | 180 | 180 | 185 | 180 | 185 | 185 |
| pH | | | | | | |
| 1 Day after preparation | 4.30 | 4.30 | 4.31 | 4.31 | 4.30 | 4.31 |
| 7 Days after preparation | 4.20 | 4.25 | 4.25 | 4.26 | 4.25 | 4.26 |
| Acidity (lactic acid %) | | | | | | |
| 1 Day after preparation | 0.98 | 0.99 | 0.98 | 0.97 | 0.99 | 0.98 |
| 7 Days after preparation | 1.05 | 1.00 | 1.01 | 1.01 | 1.00 | 1.01 |
| Flavor ¹ (point) | | | | | | |
| 1 Day after preparation | 9 | 9 | 9 | 9 | 9 | 9 |
| 7 Days after preparation | 8 | 9 | 9 | 9 | 9 | 9 |
| Curd tension² (g) | | | | | | |
| 1 Day after preparation | 45 | 44 | 46 | 47 | 43 | 45 |
| 7 Days after preparation | 50 | 51 | 48 | 50 | 53 | 49 |
| Color/Glossiness | | | | | | |
| 1 Day after preparation | Normal | Normal | Normal | Normal | Dull | Dull |
| 7 Days after preparation | Normal | Normal | Normal | Slightly Dull | Dull | Dull |

| | | | | | | |
|---|---|---|---|---|---|---|
| 1) Results of the organoleptic test by the 10-point method (Refer to Table 4 for organoleptic quality standard). The criterion point for delivery is 7 or more. | | | | | | |
| 2) Nakamura curd tension meter. The criterion for delivery is 30 g or more. | | | | | | |

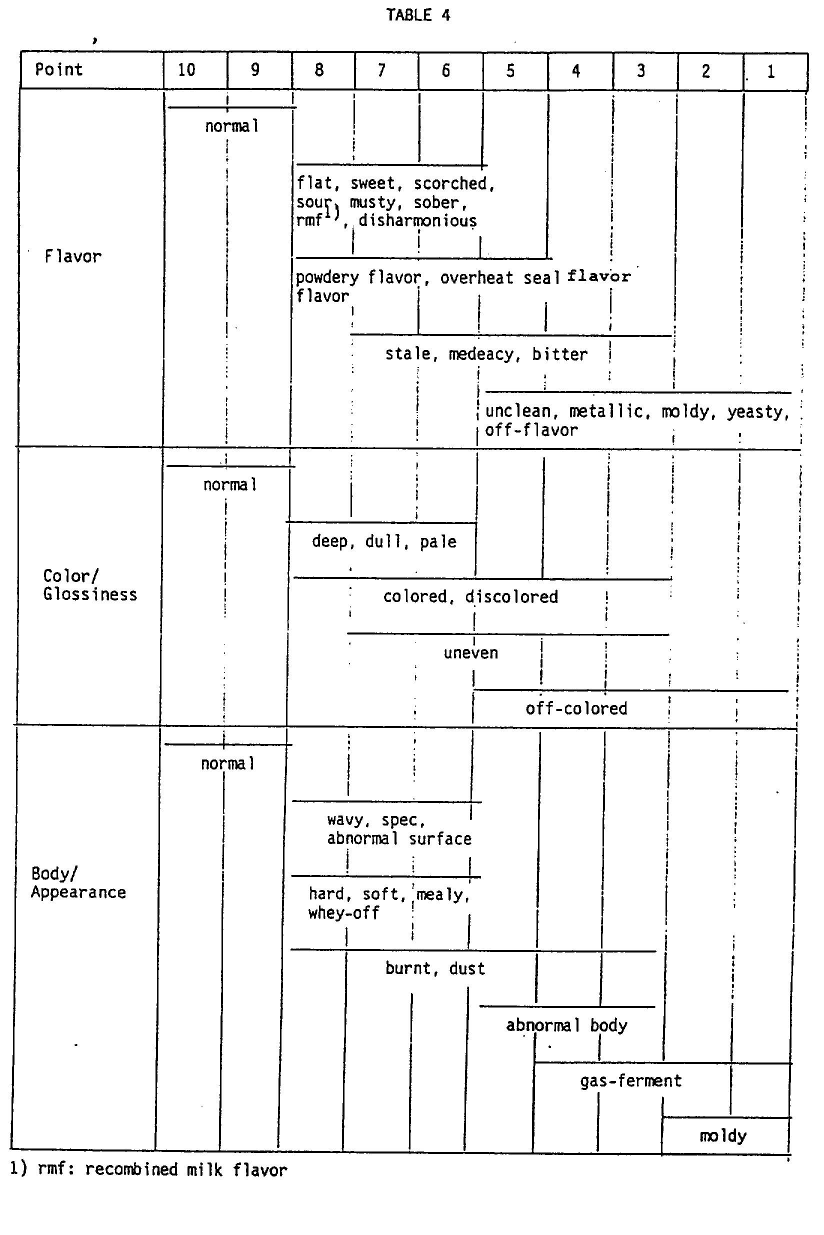

As can be seen from Table 3, there were no differences between samples (2)-(6), in which PLO was transferred from the starter, and the control (1), to which no LPO was added, in the pH, acidity, flavor, and body on the following day (1 day) after the preparation. With respect to color and glossiness, on the other hand, samples (5) and (6) having a high LPO concentration exhibited a dull color tone. On the 7th day after the preparation, however, samples (2)-(6) exhibited a higher pH, lower acidity, and better flavor than sample (1).

No differences were seen between the time required for samples to which LPO was added to achieve the acidity of the final product (lactic acid acidity: 0.85%) and the time required for the sample to which no LPO was added. Thus, the fact was confirmed, in the same way as in Example 1, that samples to which LPO was added can be handled in the same manner as normal yoghurt starters.

### [Example 4] E. coli sterilizing effect in fermented foods

25 Macacoes (age: 4-10 years) divided into 5 groups, each group consisting of 5 animals, were preliminary fed with an infant formula preparation (P7a, 13% w/w, a product of Snow Brand Milk Products Co., Ltd.) for 2 weeks, following which the infant formula preparation to which *E. coli* ATCC 25992 at a concentration of 10¹⁰ CFU/50 ml was added, was orally administered. After this, 100 ml of LPO-free yoghurt (1) prepared in Example 3 was administered every day. After 1 week, feces were collected to measure the viable cell count of *E. coli* contained therein. Then, animals were fed again only with the infant formula preparation for 2 weeks to completely restore their conditions, followed by the administration of 10¹⁰ CFU/50 ml of *E. coli* ATCC 25992 in the same manner as before. After the administration, Group A animals were fed with LPO 5 mg/l yoghurt (2), Group B with LPO 10 mg/l yoghurt (3), Group C with LPO 100 mg/l yoghurt (4), Group D with LPO 500 mg/l yoghurt (5), and Group E with LPO 1000 mg/l yoghurt (6), all prepared in Example 2, each in an amount of 100 ml. After 1 week, feces were collected to count the viable cell number of *E. coli* contained therein. The results, shown in Table 5, are expressed by the survival rate (%), the proportion of the number of *E. coli* in feces after the administration of each sample to the number of *E. coli* in feces after the administration of the control sample (1).

**TABLE 5**

| Group | Bacteria detected after administration of sample (1) (CPU) * | Bacteria detected after administration of each sample (CPU) * | Survival rate (%) |
|---|---|---|---|
| A (LPO 5 mg/l) | 3.5x10⁶ | 3.3x10⁶ | 94 |
| B (LPO 10 mg/l) | 2.8x10⁶ | 1.2x10⁶ | 43 |
| C (LPO 100 mg/l) | 6.9x10⁵ | 7.6x10⁴ | 11 |
| D (LPO 500 mg/l) | 9.1x10⁵ | 5.4x10⁴ | 6 |
| E (LPO 1,000 mg/l) | 1.7x10⁶ | 8.5x10⁴ | 5 |

| | | | |
|---|---|---|---|
| * The mean value of CFU (Colony Forming units)/g in feces | | | |

As clear from the above results, although the effect is hardly exhibited at LPO 5 mg/l, 50% or more were killed at 10 mg/l, and 90% or more at 100 mg/l. However, the effects were practically almost the same at 500 mg/l and 1,000 mg/l.

### [Example 5] Preparation of sour cream

7,000 g of 45% milk fat was warmed and homogenized, and filled in 6 stainless steel incubators with a volume of 3 l, in an amount of 970 g per incubator. The cream was sterilized and kept at 90°C for 10 minutes and cooled to 35°C. Six lactic acid bacterium starters prepared in the same manner as in Example 2 were inoculated to 6 incubators; one starter in an amount of 30 g to one incubator. After cultivation at 35°C for 4 hours, the culture products were cooled to produce 6 sour creams.

These sour creams were tested in the same manner as in Example 1. The results are shown in Table 6.

**TABLE 6**

| | Control | Lactoperoxidase Added | | | | |
|---|---|---|---|---|---|---|
| | (1) | (2) | (3) | (4) | (5) | (6) |
| LPO Content (mg/l) | 0 | 5.01 | 9.89 | 97.5 | 501.3 | 989.8 |
| Acidity (Lactic acid %) | 0.68 | 0.68 | 0.68 | 0.65 | 0.64 | 0.64 |
| Flavor ¹ | 9 | 9 | 9 | 9 | 9 | 9 |

| | | | | | | |
|---|---|---|---|---|---|---|
| 1) Results of the organoleptic test by the 10-point method (Refer to Table 4). The standard point for delivery is 7 or more. | | | | | | |

The above results show that creams to which LPO was added exhibited lactic acid acidities and flavors equivalent to the control group, demonstrating that the addition of LPO does not affect the product quality.

### [Example 6] Preparation of Quark

120 g of skim milk powder was dissolved into 870 g of water, heated and held at 95°C for 30 minutes to sterilize, and cooled to 28°C. To this was inoculated 10 g of a commercial *L. cremoris* skim milk culture (Hansen Co.) and the mixture was incubated at 28°C for 18 hours. 4 portions of the culture were provided; one without the addition of PO (1), and to the others horseradish PO was added in an amount of (2) 5 g, (3) 10 g, and (4) 20 g. They were cooled after stirring to prepare 4 lactic acid bacterium starters for the preparation of quark.

Then, 30 kg of skim milk was heated at 97°C for 15 seconds to pasteurize and cooled to 28°C. 5 kg of the cooled skim milk was charged into a sterilized 10 l stainless steel incubator. After the addition of 5 mg of rennet and 1% of one of the above lactic acid bacterium starters, the milk was cultivated at 28°C for 16 hours, then cooled with stirring. Whey was removed using a cloth bag to obtain 1,150 g of quark. 3 other kinds of quark were prepared in the same manner using 3 other lactic acid bacterium starters.

The results of measurement of horseradish PO contents and qualities of the products are shown in Table 7.

**TABLE 7**

| | Control | Horseradish PO added | | |
|---|---|---|---|---|
| | (1) | (2) | (3) | (4) |
| PO Content (mg/l) | 0 | 10 | 23 | 42 |
| pH | 4.24 | 4.28 | 4.27 | 4.28 |
| Flavor ¹ | 9 | 9 | 9 | 9 |
| Body/Texture | Smooth | Smooth | Smooth | Smooth |

| | | | | |
|---|---|---|---|---|
| 1) Results of the organoleptic test by the 10-point method (Refer to Table 4). The criterion point for delivery is 7 or more. | | | | |

The above results show that quark to which LPO was added exhibited similar pHs, flavors, and body and texture equivalent to those of the control group, demonstrating that the addition of PO does not affect the product quality.

### [Example 7]

720 g of skim milk powder was dissolved into 5,220 g of water, heated and held at 90°C for 20 minutes to sterilize, and cooled to 37°C. To this was inoculated 60 g of a commercial mixed starter consisting of *L. bulgaricus* and *S. thermophilus* (Hansen Co.) and cultivated for 6 hours. 5 lactic acid bacterium starters for yoghurt preparation were prepared from each 983.2 g portion of this cultured material; one without the addition of PO (1), and the others with the addition of 5% LPO solution, in which LPO was dissolved and dispersed in sterilized water, in a LPO amount of (2) 33.3 mg/kg, (3) 100 mg/kg, (4) 166.67 mg/kg, and (5) 333.34 mg/kg.

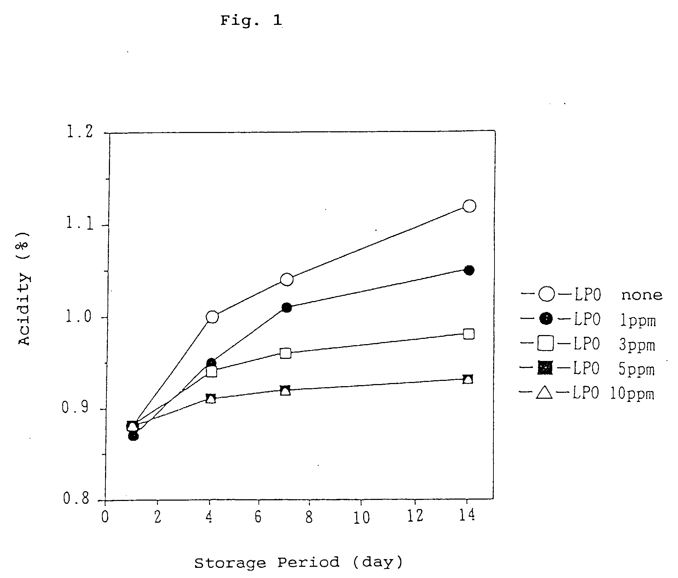

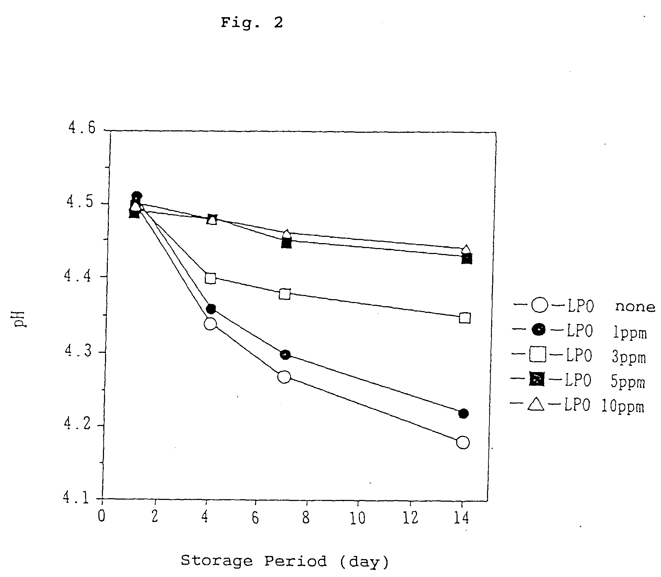

9,700 g of raw milk (a commercial milk) standardized to 3.5% milk fat was maintained at 90°C for 10 minutes to sterilize, and cooled to 40°C. The LPO concentration and thiocyanate content of this milk were measured to find that both were under the detectable limits (LPO < 25 ng/ml, thiocyanate < 0.1 ppm). One of the above lactic acid bacterium starters (300 mg) was inoculated to the milk filled in a 500 ml plastic container, and cultivated at 40°C until 0.85% lactic acid acidity was achieved, immediately followed by cooling to prepare a plain yoghurt. In this manner, 5 plain yoghurts with different LPO contents were prepared using starters with different LPO contents. These plain yoghurts were maintained at 10°C in a temperature-controlled room to measure their acidity and pH on 1, 4, 7 and 14 days after the preparation. The results are shown in Figures 1 and 2.

Since no sweeteners were added to plain yoghurts, they were very sour when their acidity exceeds 1.1. As shown in Figure 1, the LPO concentration of 1 ppm in the product could exhibit the effect of suppressing the acidity increase, but the increase during distribution period could be controlled to below 0.1% when the concentration was kept at 3 ppm or more. Thus, the adjustment of the final acidity level was confirmed to provide yoghurts with a suitable sour taste throughout the relish period.

### [Example 8]

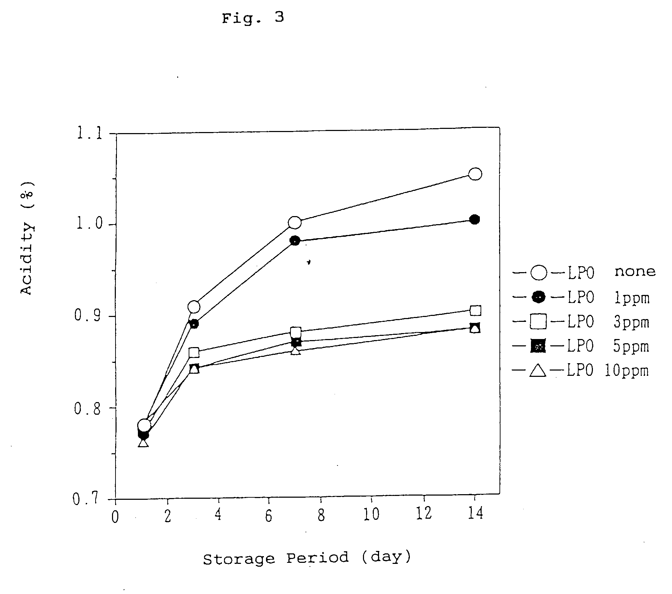

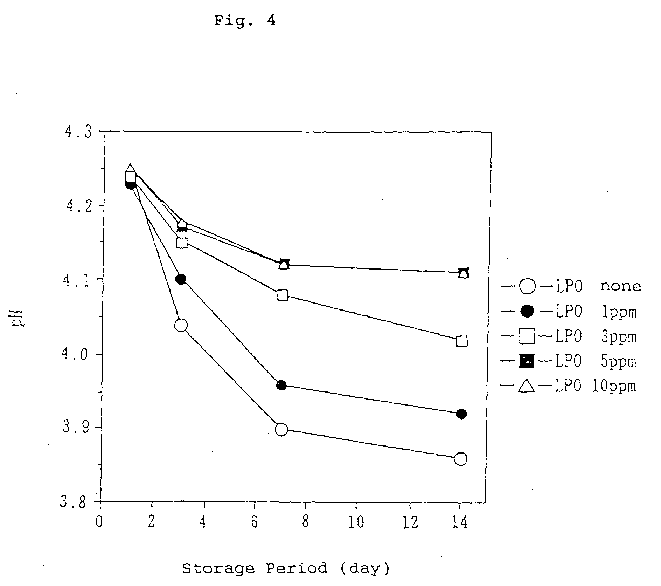

To a mixture of 4,000 g of raw milk (a commercial milk) and 1,144 g of water, which had been heated to about 60°C in advance, 900 g of sugar, 436 g of skim milk powder, and 114 g of raw cream, were added, mixed, dissolved and homogenized. To this was added a mixture consisting of 10 g of gelatin, 10 g of agar, and 5 g of low methoxylpectin dispersed in 2,993 g of water and heated to dissolve, followed by a further addition of 10 g of a yoghurt flavor. The resulting mixture was mixed, heated and held at 90°C for 10 minutes to pasteurize, and cooled to 38°C. The measurement of the LPO and thiocyanate content revealed that both were below the detectable limits. To 1,746 g portions of the raw material mixture thus obtained were inoculated 54 g of lactic acid bacterium starters to which LPO was added, prepared in the same manner as in Example 1. The mixture was fermented at 38°C until 0.65% acidity was achieved, immediately followed by cooling to prepare sweet yoghurts with LPO concentrations of 0, 1, 3, 5, and 10 mg/l. These yoghurts were stored at 10°C for 2 weeks to measure their acidity and pH. As a result, as shown in Figures 3 and 4, the acidity increase in the yoghurt to which no LPO was added was 0.27% in 2 weeks, while yoghurts to which LPO was added, even in the case of the 1 ppm concentration, exhibited the effect of controlling the acidity increase, and with 3 ppm or more the acidity increase was suppressed to about 0.1% in 2 weeks, demonstrating a remarkable control of acid production by lactic acid bacteria.

### [Example 9]

345 g of skim milk powder and 15 g of yeast extracts were dissolved in 2,530 g of water. The mixture was heated and held at 95°C for 30 minutes to sterilize, and cooled to 32°C. 45 g of *S. thermophilus* SBT-0187 and 45 g of *L. acidophilus* SBT-2062 were inoculated and cultivated at 32°C for 16 hours. Five lactic acid bacterium starters for drink yoghurt preparation were prepared from each 497 g portion of this cultured material; one without the addition of LPO (1), and the others with the addition of 5% LPO solution, in which LPO was dissolved and dispersed in sterilized water, in a LPO amount of (2) 15.4 mg/kg, (3) 46.2 mg/kg, (4) 77.0 mg/kg, and (5) 153.9 mg/kg.

1,985 g of skim milk powder, 150 g of salt-free butter, and 10 g of yeast extracts were dissolved in 12,105 g of water. The mixture was heated at 60°C and homogenized, then heated and held at 90°C for 5 minutes to pasteurize, followed by cooling to 40°C. The LPO concentration and thiocyanate content of the mixture were measured to find that both were below the detectable limits. 2,375 g of this mixture was charged into 5 stainless steel incubators and either one of the above lactic acid bacterium starters (125 mg) was inoculated to effect fermentation at 40°C until 1.2% lactic acid acidity was achieved, immediately followed by cooling to 10°C.

1,346 g of a mixture of sugar, stabilizers, and flavors, which had been sterilized and cooled in advance, was added to 2,500 g of these fermented products, and the mixture was homogenized and filled to produce drink yoghurts with different LPO contents; 0, 1, 3, 5, and 10 mg/l. These drink yoghurts were subjected to a storage test at 10°C for 14 days to evaluate the flavor, acidity, and pH of the products.

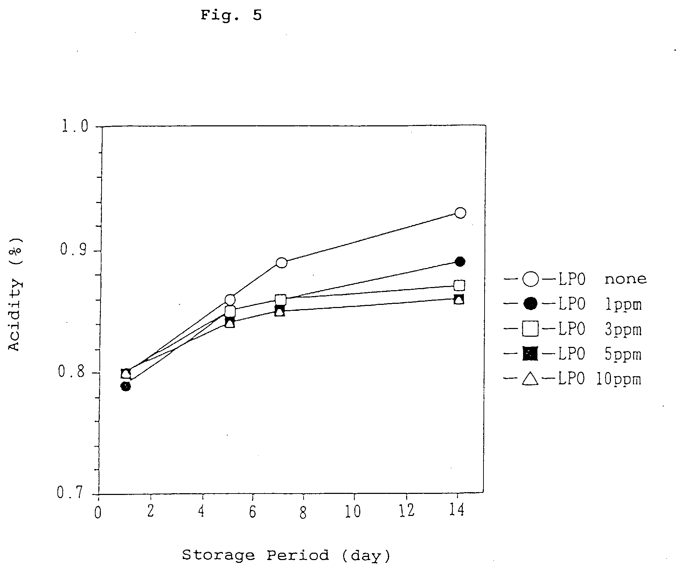

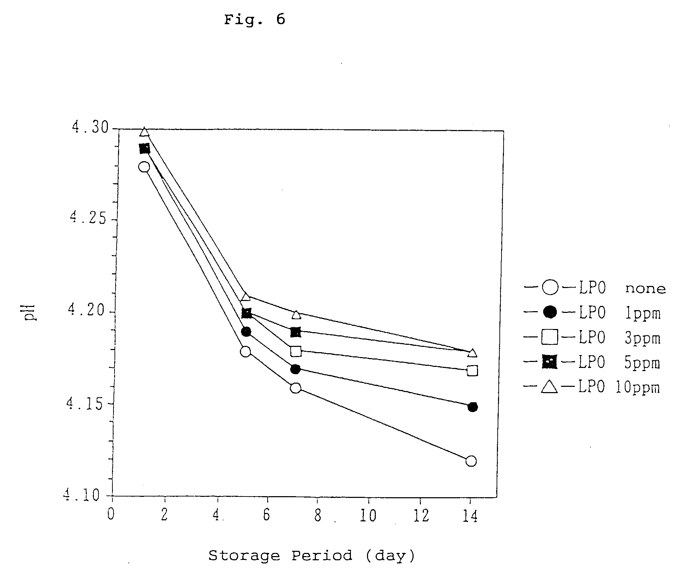

As a result, as shown in Figures 5 and 6, there were distinct differences in the acidity increase depending on the differences in LPO concentrations. The LPO concentration of 1 mg/l in the product was found to suppress the acidity increase as compared to the control, but its effects were remarkable at the concentration of 3 ppm or more. With respect to the flavor, the products to which LPO was added were evaluated to exhibit no excessive sour taste as compared with normal products to which no LPO was added; they were more flat and easy to drink. The results of the evaluation reflected Figures 5 and 6.

### Industrial Applicability

According to the present invention, PO can be homogeneously dispersed in fermented milk products in an active state without affecting the product qualities, thus remarkably promoting the effect of fermented products on the control of intestinal functions.

In addition, since growth of extraneous bacteria, such as *E. coli* or the like, attached to PO, is inhibited by lactic acid bacteria in the starter and, further, these extraneous bacteria can be killed in line with the growth of lactic acid bacteria, the products obtained is hygienic.

Furthermore, because of control of excessive increase in the product acidity throughout the storage period according to the present invention, the products may retain a proper acid taste over the entire relish period.

Consequently, if the products are manufactured by a process and under the circumstances in which contamination of microorganisms is adequately protected, a normal 2-week relish period can be extended even longer.

## Claims

1. A process for preparing edible fermented milk products which have a suppressed acidity increase during storage or an enhanced effect of controlling intestinal functions; the method comprising the following steps: i) adding peroxidase to lactic acid bacterium culture to provide a starter; ii) adding the starter to milk; and iii) fermenting the milk and starter mixture.

2. A process according to claim 1 wherein the milk is sterilised prior to adding the starter.

3. A process according to claim 1 or 2 wherein the milk does not substantially contain peroxidase and thiocyanate.

4. A process according to any preceding claim wherein the milk is raw milk.

5. A process according to any preceding claim wherein the peroxidase concentration in the fermented milk product is 10 mg/l or more.

6. A process according to any preceding claim wherein the peroxidase concentration in the fermented milk product is 1 mg/l or more.

## Patentansprüche

1. Verfahren zur Herstellung von eßbaren Produkten auf der Basis von fermentierter Milch, die eine unterdrückte Aciditätssteigerung während der Lagerung oder eine verstärkte Wirkung der Steuerung von Darmfunktionen aufweisen; wobei das Verfahren die folgenden Schritte umfaßt:
i) Zugabe von Peroxidase zu einer Milchsäurebakterienkultur, um einen Säurewecker bereitzustellen; ii) Zugabe des Säureweckers zu Milch; und iii) Fermentierung des Milch- und Säureweckergemisches.

2. Verfahren nach Anspruch 1, wobei die Milch vor Zugabe des Säureweckers sterilisiert wird.

3. Verfahren nach Anspruch 1 oder 2, wobei die Milch im wesentlichen keine Peroxidase und kein Thiocyanat enthält.

4. Verfahren nach einem der vorstehenden Ansprüche, wobei die Milch rohe Milch ist.

5. Verfahren nach einem der vorstehenden Ansprüche, wobei die Peroxidasekonzentration in dem Produkt auf der Basis von fermentierter Milch mindestens 10 mg/l beträgt.

6. Verfahren nach einem der vorstehenden Ansprüche, wobei die Peroxidasekonzentration in dem Produkt auf der Basis von fermentierter Milch mindestens 1 mg/l beträgt.

## Revendications

1. Procédé de préparation de produits laitiers fermentés comestibles qui présentent une augmentation de l'acidité empêchée pendant le stockage ou qui présentent un effet accru de la régulation des fonctions intestinales; le procédé comprenant les étapes suivantes consistant à: i) ajouter de la peroxydase à une culture bactérienne productrice d'acide lactique pour fournir un ferment; ii) ajouter le ferment au lait; et iii) fermenter le mélange de lait et de ferment.

2. Procédé selon la revendication 1, dans lequel le lait est stérilisé avant l'addition du ferment.

3. Procédé selon la revendication 1 ou 2, dans lequel le lait ne contient pratiquement pas de peroxydase ni de thiocyanate.

4. Procédé selon l'une quelconque des revendications précédentes, dans lequel le lait est du lait cru.

5. Procédé selon l'une quelconque des revendications précédentes, dans lequel la concentration en peroxydase du produit laitier fermenté est de 10 mg/l ou plus.

6. Procédé selon l'une quelconque des revendications précédentes, dans lequel la concentration en peroxydase du produit laitier fermenté est de 1 mg/l ou plus.
